**Europäisches Patentamt**

**European Patent Office**

**Office européen des brevets**

(11) Veröffentlichungsnummer : **0 451 435 B1**

(19)

(12) **EUROPÄISCHE PATENTSCHRIFT**

(45) Veröffentlichungstag der Patentschrift :
**06.10.93 Patentblatt 93/40**

(21) Anmeldenummer : **90890107.7**

(22) Anmeldetag : **09.04.90**

(51) Int. Cl.⁵ : **B27K 3/52,** B27K 3/50,
B27K 3/10, G01N 33/46,
G01N 3/42

(54) **Mittel zum Imprägnieren von Holz.**

(43) Veröffentlichungstag der Anmeldung :
**16.10.91 Patentblatt 91/42**

(45) Bekanntmachung des Hinweises auf die
Patenterteilung :
**06.10.93 Patentblatt 93/40**

(84) Benannte Vertragsstaaten :
**AT CH DE DK ES FR GB LI SE**

(56) Entgegenhaltungen :
**AT-B- 122 507**
**AT-B- 164 039**
**DE-C- 395 761**
**FR-A- 1 130 587**
**US-A- 4 220 688**

(73) Patentinhaber : **Haltmeier, Georg, Dipl.-Ing.**
**Preindlgasse 2**
**A-1130 Wien (AT)**

(72) Erfinder : **Haltmeier, Georg, Dipl.-Ing.**
**Preindlgasse 2**
**A-1130 Wien (AT)**

(74) Vertreter : **Beer, Manfred, Dipl.-Ing. et al**
**Lindengasse 8**
**A-1070 Wien (AT)**

**Beschreibung**

Die Erfindung betrifft ein Mittel zum Imprägnieren von Holz.

Es ist bekannt, daß man Holz, wie Holzmaste, Rundhölzer, Grubenholz u. dgl. durch Imprägnieren mit dem Ziel behandeln kann, die Brennbarkeit herabzusetzen, die Wasseraufnahmefähigkeit von Holz zu verringern und insbsondere das Holz gegen Schädlingsbefall zu konservieren.

Das Imprägnieren von Holz kann durch Impfimprägnieren erfolgen, das hauptsächlich angewendet wird, um beispielsweise bei Holzmasten, die besonders beanspruchte Einbauzone zu konservieren. Das Impfimprägnieren wird mit von Hand aus oder maschinell in das Holz eingetriebenen Impfnadeln ausgeführt. In diesem Zusammenhang kann auf die AT-B-173 354, 255 111 und 352 993 verwiesen werden.

Neben dem geschilderten Impfimprägnieren ist es auch bekannt, Holz unter Verwendung flüssiger Imprägniermittel, z.B. wässeriger Lösungen von Schutzsalzen nach dem Kesselimprägnierverfahren zu behandeln. Derartige Kesselimprägnierverfahren sind aus der DE-C-902 787, 967 393 und der AT-B-352 992 bekannt. Aus der AT-B-386 153 ist im übrigen ein Verfahren bekannt, bei dem eine Kombination aus Kesselimprägnieren und Impfimprägnieren angewendet wird.

Es sind schon viele Mittel zum Imprägnieren von Holz vorgeschlagen worden, in welchem Zusammenhang beispielhaft auf die AT-B-91 990, 122 507, 164 039, 177 559, die DE-C-714 105, die GB-A-141 728 sowie die CH-A-453 663 verwiesen werden kann. Die meisten bekannten Imprägniermittel enthalten in kleineren oder größeren Anteilen hochgiftige Substanzen, wie Kadmiumverbindungen oder Chromverbindungen, so daß die Anwendung der bekannten Mittel nicht immer unbedenklich ist und jedenfalls unter entsprechenden Schutzmaßnahmen zu erfolgen hat.

US-A-4 220 688 beschreibt einen Prozess zum Imprägnieren von Holz bei dem zunächst im 1.Schritt das Holz mit Tanninlösung (5-50 Gew%) imprägniert wird und im 2.Schritt mit einer 1-50 Gew-%igen Lösung eines Metallsalzes imprägniert wird.

Der Erfindung liegt die Aufgabe zugrunde, ein Mittel zum Imprägnieren von Holz anzugeben, das universell einsetzbar ist und ohne ausgesprochen giftige Wirkbestandteile zusammengesetzt ist, ohne an Konservier- und Holzschutzwirkung zu verlieren.

Erfindungsgemäß wird diese Aufgabe mit einem Mittel zum Imprägnieren von Holz gelöst, das als Wirkbestandteil ein Gemisch aus Tannin ($C_{76}H_{52}O_{46}$), 2-Hydroxybenzolcarbonsäure und Benzolcarbonsäure und zwar in Mengen von bis zu 90 Gew.-% Tannin, bis zu 40 Gew.-% 2-Hydroxybenzolcarbonsäure, bis zu 40 Gew.-% Benzolcarbonsäure, sowie gegebenenfalls bis zu 50 Gew.-% Natriumfluorid und weiters Verdünnungs- und/oder Bindemittel enthält.

Vorteilhafte Ausgestaltungen und bevorzugte Zusammensetzungen des Mittels nach der Erfindung sind Gegenstand der Unteransprüche 2 bis 5 und 7.

Als Bindemittel kann das efindungsgemäße Mittel ein solches auf Basis von Zellulose (z. B. Carboxymethylzellulose) und/oder auf Basis eines Silikats (z.B. Aluminiumsilikat) enthalten, wobei das Bindemittel in einer Menge von 2 bis 10 Gew.-% bezogen auf das Gemisch aus Wirkbestandteilen vorliegt. Bevorzugt ist ein Gehalt an Bindemittel von 5 Gew.-%. Ein derartige Bindemittel enthaltendes Mittel wird mit besonderem Vorteil zum Impfimprägnieren von Holz verwendet.

In einer Variante enthält das Mittel, insbesondere jenes nach den Unteransprüchen 3 oder 4 als Verdünnungsmittel Wasser in einer Menge von 90 bis 99, vorzugsweise 96 bis 98 Gew.-% bezogen auf das Gemisch aus Wirkbestandteilen. Dieses Mittel wird bevorzugt und mit Vorteil beim Kesselimprägnieren von Holz verwendet.

Nachstehend werden in Form einer Tabelle Beispiele für Zusammensetzungen von erfindungsgemäßen Mitteln zum Imprägnieren von Holz angegeben:

Wirkbestandteile (in Gew.-%):

| Beispiel Nr. | N | T | G | S | B |
|---|---|---|---|---|---|
| 1.1. | | 20 | | 40 | 40 |
| 2. | | 30 | | 30 | 40 |
| 3. | | 40 | | 20 | 40 |
| 2.1. | 25 | 25 | | 25 | 25 |
| 2. | 30 | 20 | | 20 | 30 |
| 3. | 40 | 40 | | 10 | 10 |
| 3.1. | | 60 | | 20 | 20 |
| 2. | | 80 | | 10 | 10 |
| 3. | | 90 | | 5 | 5 |

N: Natriumfluorid

T: Tannin ($C_{76}H_{52}O_{46}$)

S: 2-Hydroxybenzolcarbonsäure

B: Benzolcarbonsäure

Die Beispiele 1.1. bis 1.3. sind insbesondere für das Imprägnieren nach dem Impfstichverfahren geeignet und werden durch entsprechende Zumischung von Bindemitteln auf Basis von Zellulose und/oder einem Silikat zur Pastenform zubereitet.

Die Mittel gemäß den Beispielen 1.1. bis 1.3. eignen sich für das Impfimprägnieren und das Nachimprägnieren von Holz, wobei die Wirkbestandteilkombinationen der Beispiele 1.1. bis 1.3. besonders geeignet sind, wenn bei noch ausreichendem Holzschutz die Toxizität besonders niedrig sein soll. Die in den Beispielen 1.1. bis 1.3. genannte Kombination von Wirkbestandteilen ist nach nach Einstufung in einschlägigen Vorschriften (z.B. österr. Chemikaliengesetz) als nicht giftig anzusehen).

Die Wirkstoffkombinationen der Beispiele 2.1. bis 2.3. und 3.1. bis 3.3. werden bevorzugt als 2 bis 4%-ige wässerige Lösung zum Kesselimprägnieren verwendet und bewähren sich insbesondere, wenn eine niedrige Toxizität verlangt ist. Ganz besonders bewähren sich die in den Beispielen 2.1. bis 3.3. und genannten Gemische aus Wirkbestandteilen in entsprechender wässeriger Lösung (beispielsweise 2 bis 4 % Gehalt an Gemisch an Wirkbestandteilen in der wässerigen Lösung) für die Imprägnierung von Masten und Rundhölzern gemäß der AT-B-386 153.

## Patentansprüche

1. Mittel zum Imprägnieren von Holz, dadurch gekennzeichnet, daß es als Wirkbestandteil ein Gemisch aus Tannin ($C_{76}H_{52}O_{46}$), 2-Hydroxybenzolcarbonsäure und Benzolcarbonsäure und zwar in Mengen von bis zu 90 Gew.-% Tannin, bis zu 40 Gew.-% 2-Hydroxybenzolcarbonsäure, bis zu 40 Gew.-% Benzolcarbonsäure, sowie gegebenenfalls bis zu 50 Gew.-% Natriumfluorid und weiters Verdünnungs- und/oder Bindemittel enthält.

2. Mittel nach Anspruch 1, dadurch gekennzeichnet, daß es als Wirkbestandteil ein Gemisch aus 20 bis 40 Gew.-% Tannin, 20 bis 40 Gew.-% 2-Hydroxybenzolcarbonsäure und 40 Gew.-% Benzolcarbonsäure enthält.

3. Mittel nach Anspruch 1, dadurch gekennzeichnet, daß es als Wirkbestandteil ein Gemisch aus 25 bis 40 Gew.-% Natriumfluorid, 25 bis 40 Gew.-% Tannin, 10 bis 25 Gew.-% 2-Hydroxybenzolcarbonsäure und 10 bis 25 Gew.-% Benzolcarbonsäure enthält.

4. Mittel nach Anspruch 1, dadurch gekennzeichnet, daß es als Wirkbestandteil ein Gemisch aus 60 bis 90 Gew.-% Tannin, 5 bis 20 Gew.-% 2-Hydroxybenzolcarbonsäure und 5 bis 20 Gew.-% Benzolcarbonsäure enthält.

5. Mittel nach einem der Ansprüche 1 bis 4, dadurch gekennzeichnet, daß es ein Bindemittel auf Basis von Zellulose und/oder Silikat in einer Menge von 2 bis 10, vorzugsweise 5 Gew.-%, bezogen auf das Gemisch des Wirkbestandteils, enthält.

6. Verwendung des Mittels nach Anspruch 5 zum Impfimprägnieren von Holz.

7. Mittel nach Anspruch 3 oder 4, dadurch gekennzeichnet, daß es als Verdünnungsmittel Wasser in einer Menge von 90 bis 99, vorzugsweise 96 bis 98 Gew.-%, bezogen auf das Gemisch aus Wirkbestandteilen, enthält.

8. Verwendung des Mittels nach Anspruch 7 zum Kesselimprägnieren von Holz.

## Claims

1. Agent for impregnating wood, characterised in that it contains as active component a mixture consisting of tannin ($C_{76}H_{52}O_{46}$), 2-hydroxybenzenecarboxylic acid and benzenecarboxylic acid, namely in amounts of up to 90% by weight of tannin, up to 40%, by weight of 2-hydroxybenzenecarboxylic acid, up to 40% by weight of benzenecarboxylic acid and also, if need be, up to 50% by weight of sodium fluoride and, moreover, diluent and/or binding agent.

2. Agent according to claim 1, characterised in that it contains as active component a mixture consisting of 20 to 40% by weight of tannin, 20 to 40% by weight of 2-hydroxybenzenecarboxylic acid and 40% by weight of benzenecarboxylic acid.

3. Agent according to claim 1, characterised in that it contains as active component a mixture consisting of 25 to 40% by weight of sodium fluoride, 25 to 40% by weight of tannin, 10 to 25% by weight of 2-hydroxybenzenecarboxylic acid and 10 to 25% by weight of benzenecarboxylic acid.

4. Agent according to claim 1, characterised in that it contains as active component a mixture consisting of 60 to 90% by weight of tannin, 5 to 20% by weight of 2-hydroxybenzenecarboxylic acid and 5 to 20% by weight of benzenecarboxylic acid.

5. Agent according to any one of claims 1 to 4, characterised in that it contains a binding agent based on cellulose and/or silicate in an amount of 2 to 10, preferably 5%, by weight referred to the mixture of the active component.

6. Use of the agent according to claim 5 for injection impregnation of wood.

7. Agent according to claim 3 or 4, characterised in that it contains as diluent, water in an amount of 90 to 99, preferably 96 to 98% by weight referred to the mixture of active constituents.

8. Use of the agent according to claim 7 for tank impregnation of wood.

## Revendications

1. Agent pour imprégnation du bois, caractérisé en ce qu'il contient, comme constituant actif, un mélange de tanin ($C_{76}H_{52}O_{46}$), d'acide 2-hydroxybenzoïque et d'acide benzoïque, en des proportions de jusqu'à 90 % en poids de tanin, jusqu'à 40 % en poids d'acide 2-hydroxybenzoïque et jusqu'à 40 % en poids d'acide benzoïque, ainsi que, éventuellement, jusqu'à 50 % en poids de fluorure de sodium et d'autres agents diluants et/ou liants.

2.  Agent conforme à la revendication 1, caractérisé en ce qu'il contient, comme constituant actif, un mélange de 20 à 40 % en poids de tanin, 20 à 40 % en poids d'acide 2-hydroxybenzoïque et 40 % en poids d'acide benzoïque.

3.  Agent conforme à la revendication 1, caractérisé en ce qu'il contient, comme constituant actif, un mélange de 25 à 40 % en poids de fluorure de sodium, 25 à 40 % en poids de tanin, 10 à 25 % en poids d'acide 2-hydroxybenzoïque et 10 à 25 % en poids d'acide benzoïque.

4.  Agent conforme à la revendication 1, caractérisé en ce qu'il contient, comme constituant actif, un mélange de 60 à 90 % en poids de tanin, 5 à 20 % en poids d'acide 2-hydroxybenzoïque et 5 à 20 % en poids d'acide benzoïque.

5.  Agent conforme à l'une des revendications 1 à 4, caractérisé en ce qu'il contient un liant à base de cellulose et/ou de silicate, en une proportion de 2 à 10 % en poids, et de préférence de 5% en poids, par rapport au mélange de constituants actifs.

6.  Utilisation de l'agent conforme à la revendication 5 pour l'imprégnation-inoculation du bois.

7.  Agent conforme à la revendication 3 ou 4, caractérisé en ce qu'il contient, comme agent diluant, de l'eau en une proportion de 90 à 99 % en poids, et de préférence de 96 à 98 % en poids, par rapport au mélange de constituants actifs.

8.  Utilisation de l'agent conforme à la revendication 7 pour l'imprégnation du bois en cuve.